# EUROPEAN PATENT APPLICATION

(11) **EP 1 473 307 A1**
(43) Date of publication of application: **03.11.2004**
(21) Application number: 03076300.7
(22) Date of filing: 02.05.2003
(51) Int. Cl.: C08B 30/00, C07K 14/00, C12N 15/82, A01H 5/00, A23L 1/0522

(54) **Method for modifying the size and/or morphology of starch granules**

(71) Applicant: Coöperatieve Verkoop- en Productievereniging, van Aardappelmeel en Derivaten AVEBE B.A., 9641 JA Veendam (NL); Wageningen Universiteit, 6701 BH Wageningen (NL)
(72) Inventor: de Vetten, Nicolaas Clemens Maria Henricus, 9731 KH Groningen (NL); Heeres, Paul, 7876 CG Valthermond (NL)
(74) Representative: Prins, Adrianus Willem, Mr. Ir.

(57) **Abstract**

The present invention concerns the field of recombinant DNA technology in conjunction with the genetic modification of plants and concerns in particular a process for modifying the size and/or morphology of starch granules in plants by genetic engineering techniques, as well as genetically manipulated plants and plant cells themselves and recombinant polynucleotides which can be used for the genetic manipulation. The invention also concerns the starch which granule size an/or morphology is altered by expression of a polypeptide comprising at least two fused starch-binding domains.

SEQUENCE LISTING

<110> Coöperatieve Verkoop- en Productievereniing van Aardappelmeel
      en Derivaten AVEBE B.A.
      Stichting Wageningen Universiteit en Researchcentrum

<120> Method for modifying the size and/or morphology of
      starch granules

<130> P61259EP00

<140> 03076300.7
<141> 2003-05-02

<160> 5

<170> PatentIn Ver. 2.1

<210> 1
<211> 22
<212> PRT
<213> Artificial Sequence

<220>
<223> Description of Artificial Sequence: PT-rich linker
      peptide

<220>
<221> SITE
<222> (1)..(22)

<400> 1
Ser Pro Thr Pro Thr Pro Thr Thr Pro Thr Pro Thr Pro Thr Thr Pro
  1               5                  10                  15

Thr Pro Thr Pro Ser Thr
             20


<210> 2
<211> 20
<212> DNA
<213> Artificial Sequence

<220>
<223> Description of Artificial Sequence: primer

<220>
<221> misc_feature
<222> (1)..(20)

<400> 2
caacttcgag gaattctacg                                              20


<210> 3
<211> 22
<212> DNA
<213> Artificial Sequence

<220>
<223> Description of Artificial Sequence: primer

<220>
<221> misc_feature
<222> (1)..(22)

<400> 3

aagcttatgg ctgccaattc ac 22

<210> 4
<211> 17
<212> DNA
<213> Artificial Sequence

<220>
<223> Description of Artificial Sequence: primer

<220>
<221> misc_feature
<222> (1)..(17)

<400> 4
ccatggccgg agatcag 17

<210> 5
<211> 20
<212> DNA
<213> Artificial Sequence

<220>
<223> Description of Artificial Sequence: primer

<220>
<221> misc_feature
<222> (1)..(20)

<400> 5
cttctcggat ccgccaaaac 20

## Description

### FIELD OF THE INVENTION

The invention concerns the field of recombinant DNA technology in conjunction with the genetic modification of plants and concerns in particular a process for modifying the size and/or morphology of starch granules in plants by genetic engineering techniques, as well as genetically manipulated plants and plant cells themselves (including subparts of the genetically manipulated plants as well as progeny obtained by asexual or sexual propagation) and recombinant polynucleotides (DNA or RNA) which can be used for the genetic manipulation. The invention also concerns a starch of which the granule size and/or morphology is altered by a method of the present invention.

### BACKGROUND OF THE INVENTION

Starch is a plant-derived biopolymer that is formed as a granule wherein a multitude of individual starch chains is densely packed. The starch is composed of linear amylose chains and branched amylopectine chains.

Native, granular starch is insoluble in cold water. When heating a native starch in water, the starch granules swell and the starch gelatinizes to high viscosity upon reaching the characteristic gelatinization temperature. This phenomenon of gelatinisation of starch to a viscous mass of swollen granules is one of the principal characteristics by which starch is applicable in many different industries, such as the textile and food industry.

If heating of the gelatinised starch is continued, the granular organization is destroyed and granular fragments will altogether disappear. The starch thereby dissolves. The viscosity of the resulting solution is relatively low when compared to the mixture in the swelling-phase.

To alter the swelling properties of starch, the bonding between the individual starch chains can be increased by cross-linking of the starch. This generally results in reduced swelling properties.

Each starch-type has properties that are characteristic for the particular plant species by which it is formed. The properties of maize starch are known to be quite different from those of potato starch.

The size of the starch granules varies between different plants but is generally between 1-45 µm for wheat, 3-26 µm for maize and 5-100 µm for potato.

The large size of the potato granules often hampers the utilization of potato starches. Especially in sensitive applications, such as the use of starch in printing ink formulations (e.g. for prototype printing) and the use thereof, wherein potato starches may obstruct the printing apertures, the granule characteristics of starches are critical. Also chemical modification of the starch, e.g. by cross-linking, is facilitated by smaller granules since the surface-volume ratio is important for the accessibility of the modifying compound to the starch chains.

Starches are used by the food industry in a wide variety of products to provide many desired properties including viscosity, texture, mouthfeel and improved stability. Potato starch has a large swollen starch granule leaving the food dull, course and slimy. Therefore, corn or tapioca starch is used in many food application because of its small granules, leaving the food short, smooth and/or shiny.

Potato starch has, however, advantages for use in food applications in that it has a neutral taste compared to cereal starches such as corn, wheat, rice, sorghum, waxy maize and waxy sorghum, which, give food some undesirable flavour. These off-flavours have been described by some individuals as woody', corny', starchey', 'bitey' or 'chalkey', and these flavours often come out most poignant after heat treatment.

Furthermore, potato starch provides a higher viscosity than tapioca or corn starch at the same dosage.

Small granular potato starch combines the advantages of corn/tapioca starch and those of potato starch. These advantages include higher viscosity (dosage reduction), smooth and shiny texture, low level of contaminants, good processing, shelf-life stability and improved tolerance to high shear processes.

Small granular potato starches are particularly suitable for applications in soups, sauces, gravies, ready-to-serve desserts, low-fat yoghurts, bakery fillings, jams / marmalades, canned fish, canned meat, snacks, noodles and baked goods. Small granular potato starch will provide advantages over other starches, with lower starch dosage level, better taste profile, and the smooth texture normally only obtained with waxy maize or tapioca starches. The advantage of using small granular potato starch is a smooth creamy texture without excessive swollen granules. Tapioca and waxy maize provide smooth textures as they have small granules, but small granular potato starch can provide the same texture at lower starch levels.

The use of fine, small granule potato starches could also be beneficial in many non-food applications. In textile printing for instance, carboxymethylated and roll dried (amylopectine) potato starch of small granule size enables the production of finer prints and may be a valuable alternative to waxy maize starches. In the paper industry, highly cross-linked fine potato starches may replace the calibrated wheat starch with an average grain size of 10 µm in N(on) C(arbon) R(equired) paper. Furthermore, in adhesives, and particularly in highly concentrated bag adhesives, cross-linked fine potato starches may be used as a filler, and in drilling fluids, cross-linked and optionally carboxymethylated or hydroxypropylated fine starches are expected to reduce fluid loss. In addition, smaller granule potato starches may be used as filler/structurant in soap since such a starch provides a pleasant sensation to the skin.

From an industrial perspective, there is ample need for a smaller potato starch granule. In more general terms, there is a requirement for a method to modify the size and or morphology of starch granules. Such a method would be very beneficial for widening the applicability of potato starches, but is not limited thereto.

One object of the present invention is the provision of a method for the modification of the size and/or morphology of starch granules.

It is another object of the invention to provide a starch with altered granule size distribution. Other objects of the invention will become clear from the description and figures hereinbelow.

### SUMMARY OF THE INVENTION

The above objects are achieved by expressing in a starch producing plant a genetically engineered polynucleotide construct encoding a polypeptide with at least two starch-binding domains (SBDs).

It is an aspect of the present invention to provide a method for modifying the size and/or morphology of starch granules, which method comprises the production of a transgenic starch-producing plant, said plant being capable of producing a polypeptide comprising at least two starch-binding domains.

The production of a polypeptide of the invention in such a plant can suitably be accomplished by expressing a polynucleotide construct comprising encoding regions for at least two SBDs in a starch-producing plant according to a method of the invention.

Said polynucleotide is very advantageously expressed in such starch-producing plants as potato, cassava, maize or wheat and may be expressed in such parts of the plant where starch is formed, such as seeds, leaves, roots, tubers, stems, stalks, fruits, granules or flowers. Therefore, and in another aspect, the invention relates to a transgenic plant or plant part comprising a polynucleotide construct of the invention.

Surprisingly, when expressing a polynucleotide construct of the invention in a potato, the size and/or morphology of the starch granules is altered, whereas the starch itself does not exhibit a clearly altered amylose/amylopectine ratio or other characteristics. In particular, it appears that only the average size of the starch granule is reduced and the overall size distribution of the starch granules is shifted.

Therefore, and in yet another aspect, the invention relates to a transgenic plant or plant part comprising starch or starch granules of which the size and/or morphology is modified due to the expression of a polynucleotide construct of the invention in said plant. Said polynucleotide construct of the invention comprises encoding regions for at least two starch-binding domains fused or linked to each other, e.g. directly or via a suitable linker as described hereinbelow.

Upon expression of said polynucleotide in a starch-producing plant *in vivo* a polypeptide is produced, which polypeptide comprises at least two starch-binding domains that are able to bind to the starch. Without wishing to be bound by theory, the modification of the size and/or morphology of the starch granule is believed to be the result of the interaction of said polypeptide with the starch. The modifications may either be the result of the "cross-linking" action of the polypeptide of the invention or of competition for binding sites between the said polypeptide and starch modifying enzymes that normally modify the growing starch granule *in planta* and that are naturally present in said plant.

Also claimed are cloning-, expression-, and transformation vectors containing a polynucleotide construct comprising the said DNA-sequences, as well as micro-organisms containing said polynucleotide constructs.

Therefore, other aspects of the present invention include the polynucleotide and polypeptide constructs themselves. A polynucleotide construct of the invention is characterized in that it comprises encoding regions for at least two starch-binding domains, which may be the same or different, and which regions may optionally comprise a sequence encoding a linker for binding the two starch-binding domains together in a polypeptide construct of the invention.

The polypeptide construct is preferably produced in vivo after expression of the above mentioned polynucleotide constructs in plants, but may also be produced *in vitro*. The polypeptides of the invention comprise at least two starch-binding domains that are fused together, either directly or via a linker sequence.

Yet another aspect of the invention relates to the starches produced by a method of the invention. Said starches exhibit modified granule size and or granule size distribution and/or granular morphology. In a preferred embodiment, such starches exhibit or comprise fractions with reduced granule size. Such starches have numerous industrial applications. For instance a potato starch modified according to a method of the invention is a preferred substrate for derivatization processes over the wild-type potato starch.

### DESCRIPTION OF THE FIGURES

Figures 1-10 refer to the experimental part as described in Example 1.
Figure 1 shows a schematic representation of the vector PTrcHisB/(SBD)₂, used for expression of two SBDs (SBD2) or "tandem" SBD in *E. coli* and binary vector pBIN19/(SBD)₂ used for expression of a polypeptide with two SBDs (SBD2) in potato plants. In the latter case, the engineered SBD2 gene is under the control of the potato GBSS I promoter. Amyloplast entry is mediated by the potato GBSS I transit peptide.

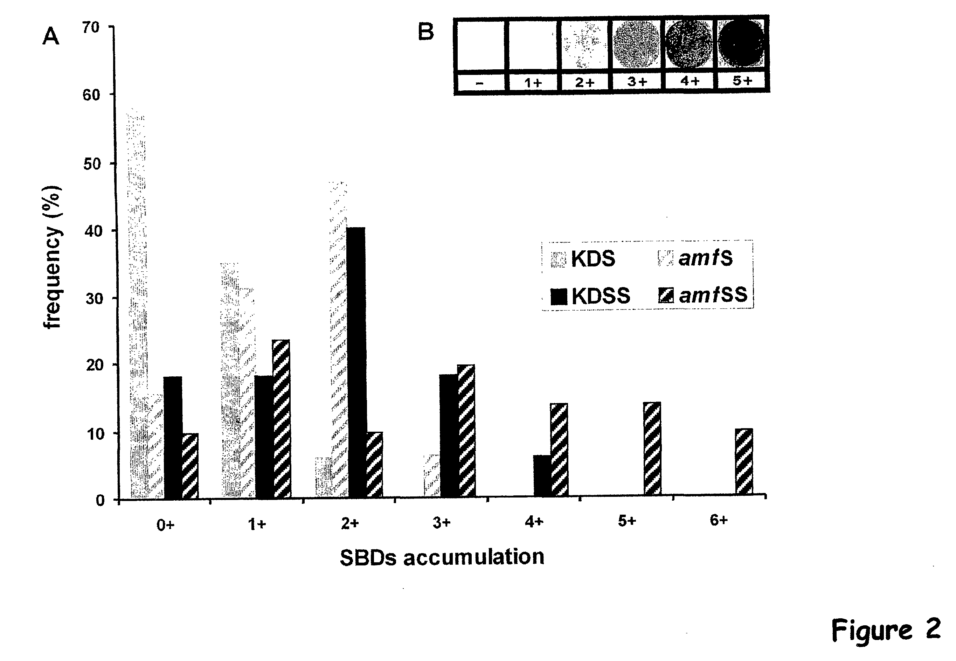

Figure 2 shows accumulation levels of SBD2 in potato starch granules isolated from amylose-containing (KD) and amylose-free (*amf*) genotypes. Panel A shows the distribution of the individual transformants over the seven classes of SBD2 accumulation in the KD and *amf* backgrounds (KDSS and *amf*SS series); for comparison, accumulation of a polypeptide with a single starch binding domain in these backgrounds is also indicated (KDS and *amf*S series). Panel B defines the classes of SBD accumulation in starch granules. This classification is based on the results of a Western dot blot analysis with the various starch samples. The 6+ category represents transgenic lines in which half the amount of starch gave a similar signal as 5+ in the Western dot blot analysis.
Figure 3 shows light and scanning electron micrographs (LM and SEM, respectively) of KD-UT (untransformed or "*wild-type*") and KDSS (amylose-containing, with double starch binding domain) starch granules. For light microscopy, the starch granules were stained with a 20x diluted Lugol solution. All starch granules were examined at a 200x magnification.
Figure 4 shows light micrographs of a potato tuber cross-section and isolated starch granules from KDSS1. Both objects were stained with a 20x diluted Lugol solution, and the images were recorded with a 200x magnification
Figure 5 shows light and scanning electron micrographs of *amf*-UT and *amf*SS transgenic starch granules. For light micrographs, the starch granules were stained with a 20x diluted Lugol solution. All images were recorded with a 200x magnification, except for the SEM of *amf*SS3, which was recorded at a 300x magnification.
Figure 6 shows high-magnification scanning electron micrographs of KDSS1 and KDSS7 starch granules. Level of magnification is indicated underneath the figure.
Figure 7 shows high-magnification scanning electron micrographs of *amf*SS3, *amf*SS23, and *amf*SS34 starch granules. Level of magnification is indicated below the figure.
Figure 8 shows examples (3+ class in figure A and 6+ class in figure B) of the granule size distribution encountered in SBD2 transgenic starches of amf potato starch. Except for amfss43 (amfss43.#01 in figure A), all double SBD transformants produce granules of smaller size than the untransformed plant (amf.#01 in figure B).
Figure 9 shows the granule size distribution (panel A) and morphology (panel B) of an *amf*SS3 starch treated with α-amylase to separate individual small granules from granule aggregates as described in the Example.
Figure 10 shows the particle size distribution of selected starches. Panel A shows the particle size distributions of *amf*-UT and *amf*SS3 starches. Panel B shows the particle size distributions of taro, wheat, cassava and waxy maize starches for comparison.

### DETAILED DESCRIPTION OF THE INVENTION

In starch-producing plants, starch is usually synthesized and present in the form of starch granules. A number of enzymes in the plant are known to interact in vivo with these granules, for instance in order to build up, modify and/or degrade the starch molecules, the starch granules and/or the structure thereof. These include enzymes such as starch synthases, branching and debranching enzymes. It is also known that some micro-organisms contain proteins/enzymes that can degrade, modify or convert starch or starch granules.

Proteins or enzymes that can interact with starch, and in particular the enzymes from micro-organisms, generally contain one or more regions that can bind to the starch and/or the starch granules. These regions are referred to in the art as "starch-binding regions", "starch-binding domains" or "starch-binding modules". For a description of some starch-binding domains that have been investigated in the art, reference is for instance made to Penninga et al. (J. Biol. Chem. 1996, 271 (51):32777-32784) and Lawson et al. (J. Mol. Biol. 1994, 236:590-600), who describe the raw starch-binding domain (E-domain) of CGTase from *B. circulans;* Sorimachi et al. (J. Mol. Biol. 1996, 259:970-987; Structure 1997, 5(5):647-661), who describe the starch-binding domain of *A.* *niger* glucoamylase; Svensson et al. (Biochem. J. 1989, 264:309-311) and Janecek and Sivcek (FEBS letters 1999, 456:119-125), who describe the sequence homology between putative starch-binding domains from a number of starch modifying enzymes in a variety of micro-organisms; Goto et al. (Appl. Environ. Microbiol. 1994, 60:3926-3930), Williamson et al. (Biochemistry 1997, 36:7535-7539) and Chen et al. (Protein Engineering 1995, 8:1049-1055), who suggest that some conserved tryptophan residues and the amino acids directly adjacent thereto may play an important role in starch-binding; WO 99/15636 wherein starch-binding domains, and in particular the so-called "D-" and "E-domain" of the maltogenic amylase from *Bacillus stearothermophilus* C599, and expression thereof in a *Bacillus* host cell is described; Chen et al. (Gene 1991, 99:121-126; Biotechnol. Prog. 1991,7:225-229), who describe a fusion of β-galactosidase and the starch-binding domain from an *Aspergillus* glucoamylase; and Dalmia et *al.* (Biotech. & Bioeng. 1995, 47:575-584), who describe fusions of β-galactosidase and the starch-binding domains of glucoamylase I of *Aspergillus awamori* and of cyclodextrin glucanotransferase (domain E of CGTase) from *Bacillus macerans,* respectively.

Sumitani *et al* (Biochem. J. 2000, 350:477-484) proposed that amylolytic enzymes are classified into three families on the basis of their SBD structure. The family I SBDs (belong to Family CBM20, http://afmb.cnrs-mrs.fr/CAZY/) are widely distributed in micro-organisms (the SWISS-PROT entry is over 50 members in this family) and contains more members than the other two families. SBD in glucoamylase from *R. oryzae* is an example of family II (Family 21, the SWISS-PROT entry is over 20 members). The family III SBDs (Family CBM25, the SWISS-PROT entry is 10 members) are found only in Gram-positive bacteria, mainly the genus *Bacillus* such as α-amylase from *Bacillus* sp. no.195. The database with carbohydrate-binding modules is continuously updated and can be viewed at the above website. SBDs used in the present invention are preferably from the family I.

The term 'starch-binding domain' or 'SBD' is well-known in the art, for instance from the references cited hereinabove, and is generally used to denote any part, region or domain of a protein or polypeptide, and in particular of an enzyme, that has natural affinity to (i.e. that binds to, attaches to, complexes with, or otherwise associates with) starch or starch granules, or more generally with polymers of glucans, in particular alpha glucans.

As such, the starch-binding domains used in the invention may be derived from any protein or polypeptide known per se that contains one or more starch-binding domains, including proteins or polypeptides derived from plants, animals, fungi, algae, yeasts, bacteria and/or other micro-organisms. Any naturally occurring starch-binding domain, or any part or fragment thereof that still has affinity for starch (granules), may be used, as well as variants or mutants thereof.

The starch-binding domains may be homologous or heterologous to the plant in which the polynucleotide construct of the invention is expressed. Preferably, a starch-binding domain of an enzyme is used, and is more preferably a domain derived from a bacterium, yeast, fungus or (other) microorganism, or of a plant.

Some non-limiting examples of enzymes from which the starch-binding domains may be derived are mentioned in the prior art indicated above, and may further include enzymes such as the cyclodextrin glycosyl transferases ("CGTases"), for instance from *Bacillus circulans, Aspergillus awamori, Aspergillus kawachi, Klebsiella pneumoniae* or *Bacillus staerothermophilus;* SBDs derived from thermostable enzymes, such as the CGTase of *Thermoanaerobacterium thermosulfurigenes;* glucoamylases, for instance from *Aspergillus niger* (which is reinforced by a disulfide bridge); glucoamylase from *Rhizopus oryzae,* alpha-amylase from *Streptomyces limosus,* beta-amylase from *Clostridium thermosulfurogenes,* maltogenic alpha-amylase from Bacillus *stearothermophilus,* maltotetraose-forming amylase from *Pseudomonas stutzeri;* as well as enzymes from plants or animals such as beta amylases.

Other suitable starch-binding domains include for instance those derived from the enzymes mentioned in US-A-5,665,585, as well as the natural starch-binding domains and variants thereof described by Penninga et al., Lawson *et al.,* Sorimachi *et al.,* Svensson *et al.,* Goto *et al.,* Williamson *et al.,* Chen *et al.* and Dalmia *et al.,* above.

Although generally, all starch-binding domains known per se can be used in the invention, including but not limited to those indicated above, it should be understood that for some applications of the invention, some starch-binding domains may be preferred compared to others. For instance, for the modification of a starch (granule), it may be that some types of starch-binding domains may direct the polypeptide construct of the invention with said at least two starch-binding domains towards the surface of the starch granule-for instance so as to alter the morphological properties of and/or associated with said surface-whereas other types of starch-binding domains may cause the polypeptide construct of the invention with said at least two starch-binding domains to be incorporated/enclosed within the starch granule.

In this way, the invention makes it possible to use a starch-altering polypeptide construct as described herein to alter the size and/or the morphology of the starch (granule), and to control, to at least some extent, where said starch-altering polypeptide construct effects its size- and/or morphology-altering activity, *e.g.* within the starch granule and/or at the surface of the starch granule.

Generally, starch-binding domains are polypeptides of about 95 to about 105 amino acids amino acids, although their size is not essential in the invention.

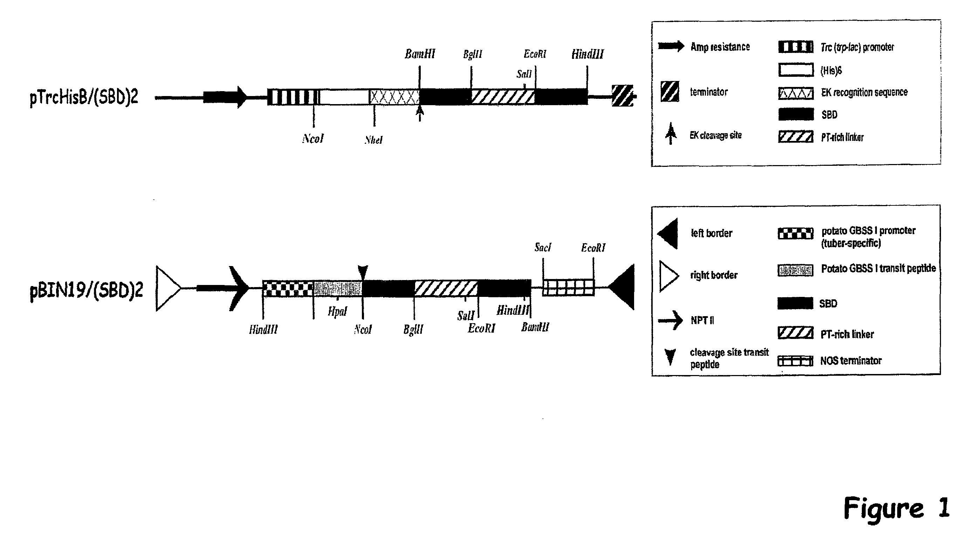

According to one embodiment, the starch-binding domains used in the invention contain at least one of the minimal sequences shown in Figure 1 of WO 00/77165, which is incorporated by reference herein, or a variant or mutant thereof, for instance in which one of the tyrosine residues in binding site 2 have been replaced by tryptophan.

Other suitable starch-binding domains can be identified by means of sequence alignment of the above minimal sequences of Figure 1 of WO 00/77165 with a known database, for instance an alignment program known per se such as BLAST or PC gene. In general, any domain or region of an enzyme that contains a sequence that has a sequence homology of more than 50%, preferably more than 70%, more preferably more than 90% with the abovementioned minimal sequence as can be used (in which a deletion or insertion is counted as a single mutation).

Preferably, any binding domain used in the invention has an affinity for starch (granules) (expressed as K_{ad}-value) of more than 10, preferably more than 15 mL/g, as measured by means of adsorption isotherm methodology as described by Chen *et al,* for instance in the Protein Engineering- and/or Gene-references mentioned above. In general, this involves measuring the adsorption of the binding domain to native starch at different concentrations of the protein (i.e. of the binding domain). After mixing the protein (for instance in concentrations ranging from 0.1 to 1.0 mg/ml) and the starch (for instance 0.1 g) in a suitable aqueous medium (for instance 1 ml total volume), the mixture is shaken for a suitable period of time (for instance 20 min to 1 hr) at a suitable temperature (for instance 4°C), after which the mixture is subjected to centrifugation (for instance 17,400 x g for 20 min), after which the protein concentration of the supernatant is assayed and the amount of adsorbed protein is determined by subtraction. Values for the adsorption constant (K_{ad}) can then be derived from the slopes of the linear adsorption isotherms, optionally by comparison with a reference protein.

With respect to the affinity for starch and the K_{ad}-value, it will further be clear to the skilled person that these may also be influenced by factors such as the type of starch used, the presence of glycosylation, the size of the construct, the type of linker (if any) etc. In addition, other assays for determining the affinity of a protein to starch have been described in the art, and these can be used to identify suitable domains or regions.

In the polynucleotide construct expressed according to the invention, the sequences encoding at least two starch-binding domains may be positioned directly adjacent to each other or with a linker sequence thereinbetween, i.e. a linker sequence of 1-100, preferably 4-60 (optionally glycosylated) amino acids that, in the resulting polypeptide, connects one starch-binding domain with one or more other starch-binding domains. The linker sequence may also act as a "hinge" and/or spacer, for instance in order to ensure that the binding of the starch-binding domain to the starch (granule) facilitates the binding of another starch-binding domain to the starch (granule).

In principle, any natural or synthetic amino acid sequence can be used as a linker, preferably a sequence that essentially does not interfere with the affinity of the starch-binding domain for the starch (granules).

Some non-limiting examples of suitable linker sequences include:
- The 40 AA linker sequence used in *Trichoderma,* comprising a flexible part and glycosylated rigid part (M. Srisodsuk, T. Reinikainen, M. Penttilä, T. Teeri (1993) Role of the interdomain linker peptide of *Trichoderma reesei* cellobiohydrolase I in its interaction with crystalline cellulose. *J. Biol. Chem.* **268**, 20756-20761(;
- Naturally occurring or synthetic sequences of alternating Pro-Thr, such as the PT-rich linker peptide derivable from exoglucanase (Cex) from *Cellulomomas fimi;*
- Linker sequences containing major amount glycine residues or of (optionally O-glycosylated) serine or threonine residues.

Other suitable linkers are for instance described by P. Argos, J. Mol. Biol. (1990), 211, p. 943-958.

PT-rich linkers seem to provide a relatively stiff connection between one starch-binding domain and one or more other starch-binding domains. If more flexible linkers are sought, such linkers as for instance occur in glucoamylase between the catalytic domain and the SBD, which linker is glycin-rich, may for example provide more flexible linkers, at least when unglycosylated.

The at least two starch-binding domains may also be bound to each other via an amino acid sequence that can be cleaved, i.e. chemically or preferably enzymatically. Examples are amino acid sequences that provide enzymatic cleavage site for enzymes such as thrombin, factor Xa, and collagenase can be mentioned. Such a sequence may also form part of a larger linker sequence as described above.

The two or more starch-binding domains in a construct of the invention may be the same or different (such as different domains from the same enzyme or domains from different enzymes/sources).

The polypeptide constructs of the invention are obtained by *in vivo* expression in a plant of a nucleotide sequence (polynucleotide construct) that codes for the polypeptide construct of the invention. For this purpose, the plant may be or may have been transformed with said polynucleotide construct, or may be a descendant (such as obtained via sexual or asexual multiplication, including crossing and/or other breeding techniques) of a plant that has been transformed with such a polynucleotide construct, and that has inherited the polynucleotide construct.

The plant may be any monocotyledonous or dicotyledonous plant in which the polynucleotide constructs can be expressed, but is preferably a plant that naturally contains or produces starch, and more preferably a plant that contains or produces starch granules, either throughout the entire plant or in any part thereof, including seeds, leaves, roots (including tuberous roots), tubers, stems, stalks, fruits, granules or flowers, and in particular the honey producing parts of flowers; and such a plant is referred to herein as a 'starch granule producing plant'. As mentioned above, said starch granules will usually be associated with or present in specific organelles of the plant cell, and in particular the plastids, such as amyloplasts, but also chloroplasts and/or chromoplasts.

Some preferred non-limiting examples of starch granule producing plants suitable for use in the invention include economically important crops such as potato, sweet potato, cassava, pea, taro, sago, yam, and/or cereals such as rice, maize, wheat and barley; of which potato, cassava, sweet potato, maize and wheat are especially preferred.

The starch granule producing plant may also be a plant, and in particular a genetically modified plant, that as such already produces a modified starch, such as transformed potato plants producing mutant amylose free ("*amf*") starch as described in e.g. CA 2061443 and WO 92/11376.

A polynucleotide construct of the invention may be obtained by 'combining' the nucleotide sequence encoding one nucleotide sequence that codes for a starch-binding domain with at least one nucleotide sequence that codes for the same or another starch-binding domain, optionally with or via one or more sequences that encode a linker sequence as described above, in such a way that expression of the combined sequences in the desired plant leads to the formation of the polypeptide construct.

Generally, this involves covalently binding the nucleotide sequences in the same reading frame and in the same orientation, and in the correct order from the 5' end to the 3' end. This can be carried out using genetic manipulation techniques known per se, such as those described in Sambrook et al,"Molecular Cloning: A Laboratory Manual" (2nd. ed.), Vols. 1-3, Cold Spring Harbor Laboratory (1989); or F. Ausubel et al, eds., "Current protocols in molecular biology", Green Publishing and Wiley Interscience, New York (1987).

The one or more sequence(s) encoding the starch-binding domains can be provided synthetically using known DNA synthesis techniques, but are preferably isolated from the organism from which the starch-binding domain has been derived (i.e. in which it naturally occurs).

The polynucleotide construct of the invention may further contain all other elements known per se for nucleic acid sequences or polynucleotide constructs, such as promoters or other control elements, terminators, translation or transcription enhancers, integration factors, signal sequences, selection markers, etc., that are preferably suited for use in (the transformation of) the host plant. The sequences that encode these further elements of the construct may again be either isolated from a suitable biological source, or provided synthetically. Examples of suitable elements are for instance described in DE-A-195 34 759, WO 91/19808, US-A-5,349,123, US-A5,750,875 and WO 92/14827.

The one or more nucleotide sequences encoding the further elements of the polynucleotide construct can again be combined with the nucleotide sequence encoding the at least two starch-binding domains in a manner known per se, such as described in Sambrook et al., Ausubel et al., DE-A-195 34 759, WO 91/19808, US-A-5,349,123 or US-A-5,750,875.

Preferably, they are combined in such a way that - after transformation - the polynucleotide construct can be used for the expression of the polypeptide construct in the desired plant. Generally, this involves combining the control elements and any further elements with the sequence encoding the polypeptide construct in an operable manner, i.e. in the same reading frame and in the same orientation, and in the correct order from the 5'end to the 3'end.

The promoter can be any promoter that is able to control/induce the expression of the polynucleotide construct in the intended plant, including constitutive and inducible promoters, and may be homologous or heterologous to said plant.

Also, a promoter may be used that directs the expression of the polynucleotide construct to a specific part or tissue of the plant, and in particular to a tissue or part of the plant where starch (granules) are formed or present, including the seeds, leaves, roots (including tuburous roots), tubers, stems, stalks, fruits, granules or flowers, and in particular the honey-producing parts of flowers, etc.. Furthermore, a promoter may be used that induces expression of the polynucleotide construct during a specific period in the life cycle of the plant. For instance, in potato, a promoter may be used that specifically directs the expression of the polynucleotide construct in or to the tuber, and/or that allows for expression only during the time the plant forms its tubers.

Examples of suitable promoters include the CaMV promoter, GBSS promoter, patatin promoter, Ubiquitin promoter, ST1 promoter, TR1 promoter, napin promoter, as well as for instance the promoters described in DE-A-195 34 759, WO 91/19808, US-A-5,349,123, US-A-5,750,875 and WO 92/14827. For specific expression of foreign genes in potato tubers, reference is made to for instance EP 0 375 092 and Rocha-Sosa et al., EMBO J. 8,23-29 (1989).

The polynucleotide construct of the invention may also comprise one or more sequences that encode signal proteins, including pre-, pro-of prepro-sequences. These usually precede the sequence encoding the polypeptide construct, such that the polynucleotide construct is expressed as a (further) polypeptide construct with these signal proteins. The signal sequence may specifically direct the expressed polynucleotide construct to a desired part or organelle within the plant or plant cell, and in particular to the starch granule(s). In particular, signal sequences for plastid targeting, such as for amyloplast, chloroplast or chromoplast targeting can be used, or signal sequences for targeting the vacuole. Some non-limiting examples thereof include the small subunit RuBisCo, GBSS transit peptides and sporamine transit peptide.

The recombinant polynucleotide constructs may be inserted into vectors, which may be commercially available, suitable for transforming into plants and suitable for expression of the polynucleotide construct in the transformed cells. Preferably used are binary vectors which are useful for plant transformation using *Agrobacterium.*

According to one preferred embodiment, the polynucleotide construct is preferably in a form suitable for transformation of a plant, such as a vector or plasmid. As such, the construct is preferably such that upon transformation it is incorporated into the (genomic) DNA of the plant. However, the polynucleotide construct may also be in any other form that can provide for expression of the polynucleotide construct in the plant, and that preferably also can be stably and/or independently maintained and/or replicated in the plant, and/or inherited from one generation of the plant to the next. The polynucleotide construct is preferably further in a form that can be stably and/or independently maintained and/or replicated in any organism to be used for constructing or selecting the polynucleotide construct and/or to be used in transforming the plant, such as *Agrobacterium.*

Although some of the embodiments of the invention may not be practicable at present, for example because some plant species are as yet recalcitrant to genetic transformation, the practising of the invention in such plant species is merely a matter of time and not a matter of principle, because the amenability to genetic transformation as such is of no relevance to the underlying concept of the invention.

Transformation of plant species is now routine for an impressive number of plant species, including both the *Dicotyledoneae* as well as the *Monocotyledoneae.* In principle any transformation method may be used to introduce chimeric DNA according to the invention into a suitable ancestor cell. Methods may suitably be selected from the calcium/polyethylene glycol method for protoplasts, electroporation of protoplasts, microinjection into plant material, (DNA or RNA-coated) particle bombardment of various plant material, infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium* mediated DNA transfer. Especially preferred is the use of the so-called binary vector technology as disclosed in EP 0 120 516 and U.S. Patent 4,940,838.

A further aspect of the invention therefore relates to a bacterium, virus or other organism suitable for transforming a plant, containing a polynucleotide construct as defined above, and preferably capable of transferring said polynucleotide construct into a plant. The organism is preferably a strain of *Agrobacterium.*

Any tissue or cell type of a plant may be transformed in a method according to the present invention.

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or (tissue) electroporation. Transgenic maize plants have been obtained by introducing the *Streptomyces hygroscopicus bar*-gene, which encodes phosphinothricin acetyltransferase (an enzyme which inactivates the herbicide phosphinothricin), into embryogenic cells of a maize suspension culture by microprojectile bombardment. The introduction of genetic material into aleurone protoplasts of other monocot crops such as wheat and barley has been reported. Wheat plants have been regenerated from embryogenic suspension culture by selecting embryogenic callus for the establishment of the embryogenic suspension cultures. The combination with transformation systems for these crops enables the application of the present invention to monocots.

Monocotyledonous plants, including commercially important crops such as rice, wheat and corn are also amenable to DNA transfer by *Agrobacterium* strains (WO 94/00977; EP 0 159 418; EP 0 856 060).

After construction, the polynucleotide construct is transformed into the desired plant, preferably a starch granule producing plant as defined above. Transformants may be obtained with or without selection.

A method for production of a transgenic plant or plant part according to the invention may comprise the step of selecting transformed plants or plant parts. Generally after transformation, plant cells or cell groupings are selected for the transfer with the polynucleotide construct comprising the DNA-sequence with the genes encoding the various enzymes or blocking mechanisms according to the invention, following by steps know to the skilled person by which the transformed material is regenerated into a whole plant and evaluating the transformed plant for the modification of starch according to the invention.

After transformation, a plant is (re) generated from the transformed cells or tissue and the polynucleotide construct is expressed in the plant or part thereof, optionally upon induction thereof in a suitable manner. Such a suitable manner may comprise the steps of transforming a plant with a polynucleotide construct as described above, such that said polynucleotide construct is expressed in the plant or at least part thereof and optionally further comprising the step of providing descendants and/or further generations of the thus transformed plant, for instance via sexual or asexual multiplication, including crossing and/or other breeding techniques.

Upon expression, the polypeptide construct will usually become 'associated with' any starch granules present in the plant, by which is meant that the polypeptide construct attaches to, binds to, complexes with or otherwise combines with the starch granule, i.e. via the at least two starch-binding domains present in the polypeptide construct. Said association may be such that the polypeptide construct is present on the surface of the starch granule, and/or incorporated into (such as by encapsulation or enclosure) the starch granule (e.g. during the biosynthesis thereof).

The association(s) thus obtained will be collectively referred to hereinbelow as the 'complex'.

As mentioned above, the starch granules in a plant will usually be present in or associated with specific organelles within the plant cell, and in particular the plastids, such as the amyloplasts, chloroplasts or chromoplasts. It should be understood that when in the present description and claims mentioned is made of a "starch granule", this is also meant to include (the starch granules as present in) these organelles.

A further aspect of the invention relates to a complex, comprising a construct with at least two starch-binding domains, associated with a starch granule.

The invention further relates to the transformed plants, or any descendant thereof, as well as cultivation material of said plant, including seed, tubers, stakes or seedlings. Usually, and preferably, the polypeptide constructs and the starch granule will already associate in vivo, so that they can be obtained/isolated together, using techniques known per se for the isolation of the starch granules from the plant or plant material. However, the invention is not limited thereto. For instance, the complex may also be formed during or as a result of the isolation/further processing of the plant or plant material, for instance when the polynucleotide construct is expressed in a part of the plant (cell) separate(d) from the starch granule.

The complex thus obtained may be processed further, for instance for further purification. The polypeptide construct may also be cleaved in a further processing step of the isolated starch granules (i.e. at a suitably situated enzymatic cleavage site as described above) in order to provide a starch wherein e.g. the linked SBDs are (partially) cleaved.

In this way, the invention can be used to provide a transformed plant that *in vivo* produces starches that are modified or altered (i.e. compared by the starch naturally produced by the original starch producing plant).

The starches obtained by using a method of the invention exhibit altered granular morphology. Also these starches exhibit size alterations as compared to untransformed and/or "*wild-type*" (WT) starches, *i.e.,* starches that can be obtained from untransformed plants. Also, the starch granules obtained from independent transformants may exhibit variations in morphology and size. The starches obtained may therefore be processed post-isolation by different techniques such as for instance sieving, whereby starch granules of a distinct size can be separated from those of other sizes.

Grains of starch of the invention often exhibit grooves on their surface, which suggests that the starch exhibits higher porosity that untransformed potato starch or other starches. This provides a starch of the invention with a better accessibility to reagents thus making it an advantageous and improved substrate for use in starch-derivatization processes and reaction. Also, as a result of the presence of the SBDs, the starches of the invention may exhibit a structure with enhanced porosity making them suitable for use in slow release applications in the pharmaceutical industry or other industrial applications relating to the use of porous carriers.

The starches produced by a method of the invention may be used in all applications where the use of small granule starches is preferred, for instance in printing ink.

The invention will now be further illustrated by means of the Example given hereinbelow.

### Example 1.

A polynucleotide comprising two SBDs (SBD2) was engineered by fusing two identical SBD sequences (i.e. the last part of the cyclodextrin glycosyltransferase gene of *Bacillus circulans;* totalling 309 nucleotides) to either end of the sequence of a PT-rich linker peptide (-SPTPTPTTPTPTPTTPTPTPST-) derived from exoglucanase (Cex) from *Cellulomonas fimi.* Thus, double SBD (or SBD2) is an artificial sequence. To our knowledge there are no such repeats with this type of SBD (a CBM20) in nature; however, a different (less well characterized) type of SBD is known to occur as double or triple repeats in particular starch degrading enzymes.

For expression of the engineered SBD2 gene comprising two SBDs (SBD2) in potato plants, the engineered SBD2 gene was ligated into the binary vector pBIN19 and was brought under the control of the potato GBSS I promoter (this promoter is used to obtain tuber-specific expression). The transit peptide which is also present in the gene product mediates the entry of the potato GBSS I transit peptide into the amyloplast. The construct is referred to as pBIN19/(SBD)2. (see Figure 1). The SBD2 polynucleotide construct was introduced in amylose-containing (KD) and amylose-free (amf) potato plants by *Agrobacterium*-mediated transformation, leading to two series of transgenic potato lines, KDSS and *amf*SS, respectively.

The amount of polypeptide construct accumulated in the potato plants as a result of the expression of the SBD2 polynucleotide construct was measured by using a Western dot blot approach and an Anti-SBD polyclonal antibody, raised by injecting a mixture of purified (denaturing conditions) *E.coli* SBD protein and SPECOL into a rabbit. The SBD2 accumulating lines (both the KDSS and the amfSS series) of the Western dot blot were grouped in 7 classes with different levels of SBD2 accumulation (ranging from 0+ to 6+). As can be seen in Figure 2, it is apparent that higher levels of SBD2 can be accumulated in an *amf* background in comparison with a KD background. This is in line with the results obtained for SBD1 constructs. The results suggest that the affinity of SBD2 for starch is higher than that of SBD1, because many more transformants are found in the classes with high SBD accumulation. Therefore, it was concluded that: (i) SBD2 is a higher-affinity binding domain than SBD1. (ii) Amylose-free backgrounds display a higher SBD2 accumulation than an amylose-containing background.

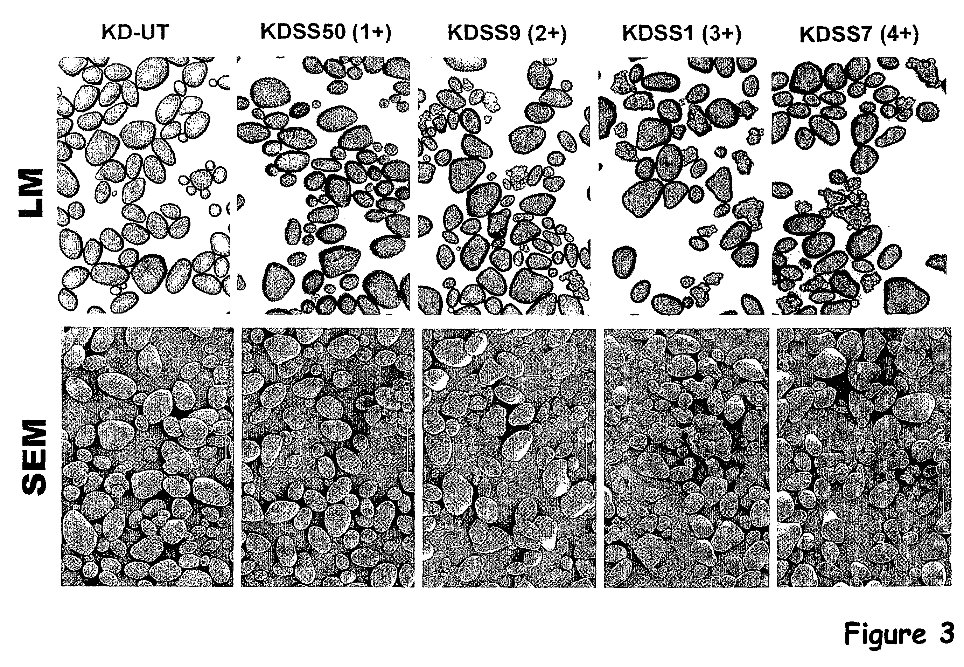

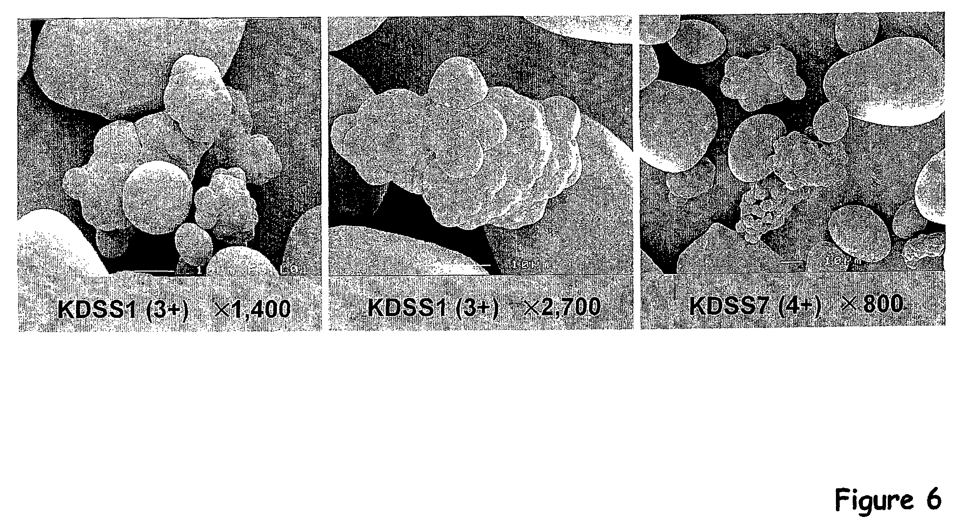

The starch granules were isolated from the various plants produced and were stained with Lugol solution and examined by light microscopy. As suggested by Figure 3 and 6, the KDSS starches, independent of the level of SBD2 accumulation, contained similar amounts of amylose as the untransformed control (KD-UT). This was further substantiated by an amylose determination of selected samples (KDSS 1, 5, 6, 7, 9, 12, 16, 19, 32, 40, 46, 50, covering the various classes of SBD2 accumulation).

In all samples the amylose content was around 20%. This suggests that GBSS I binds stronger to starch than SBD2, or that GBSS I and SBD2 bind to different locations in the granule. The morphology of KD-UT and KDSS50 (1+) is more or less similar, except for the presence of cracks in the granule surface. Such cracks are also observed with SBD1 constructs, although they seem to be less pronounced and with a lower frequency.

Further, higher accumulation levels show altered granule morphology. Granules are sometimes organized in clusters of very small granules (grape-like structures). This altered morphology appears to be most pronounced in the 4+ class (KDSS7). Figure 6 shows the grape-like structures at higher magnification. From microscopic observations it was therefore concluded that the SBD2 transformation had no effect on the amylose content of the starch and that the granular morphology was altered as a result of the transformation, the severity of which is (at least partially) proportional to the amount of SBD2 accumulated.

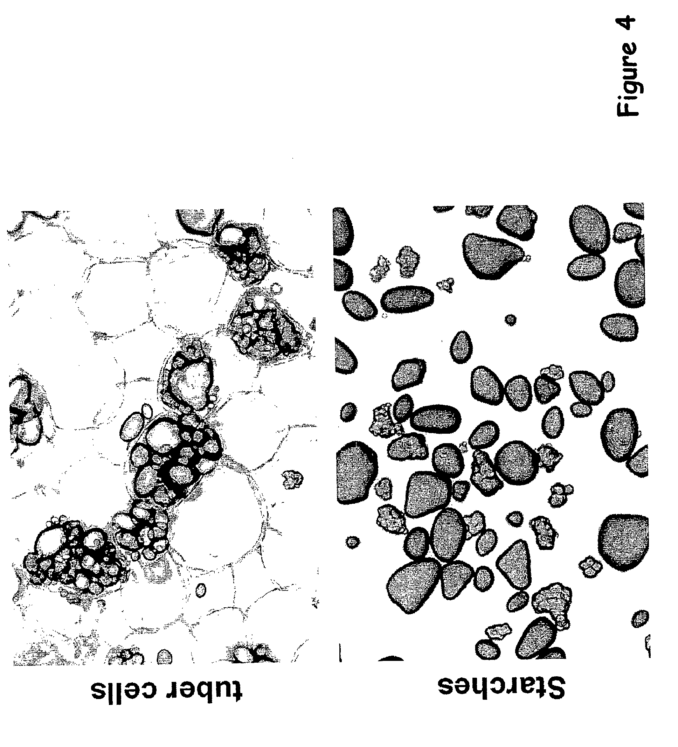

It was further investigated by light microscopy whether particular cells (or cell types) accumulate the grape-like structures. The results of these investigations are summarized in Figure 4. It was concluded that the grape-like structures were not associated with one particular cell or cell-type. Instead, they seem to occur side-by-side with normal granules within one cell.

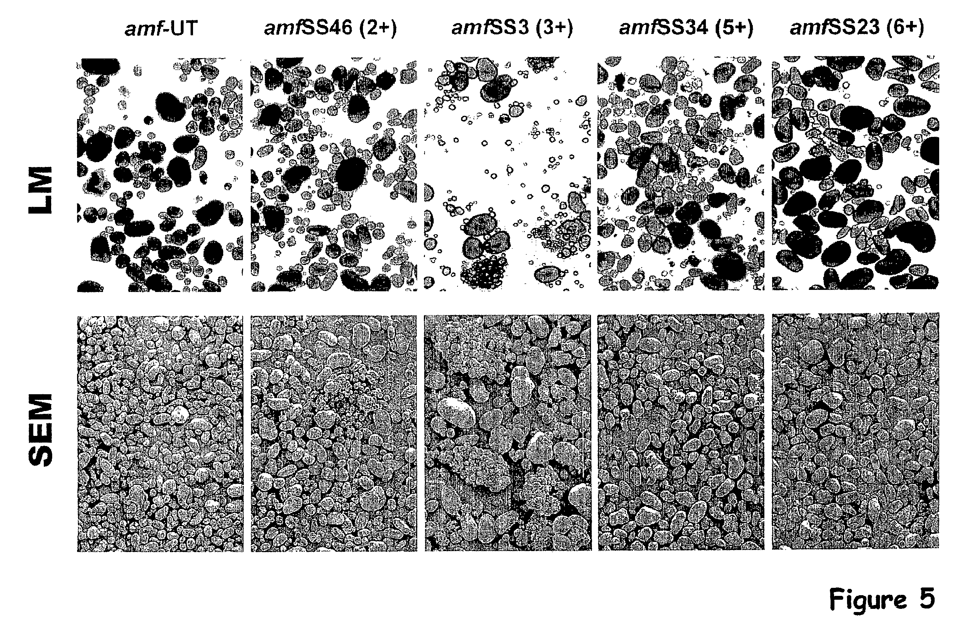

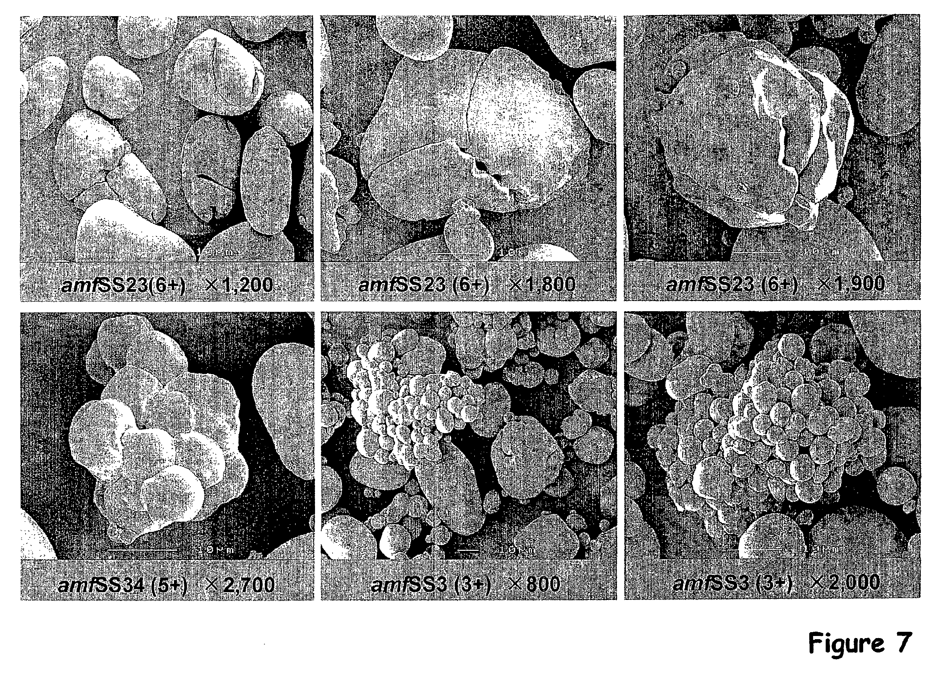

Similar investigations were carried out on potato plants of the *amf* phenotype. From microscopic observations of the *amf*SS series (as summarized in Figures 5 and 7), it was apparent that the granule size of the various transformants was not similar to that of the untransformed control. In some cases (e.g. *amf*SS46 and *amf*SS3 with relatively low levels of SBD2 accumulation) large clusters of small granules were observed. In other cases (e.g. *amf*SS34 and *amf*SS23 with the higher levels of SBD2 accumulation), the large clusters were not observed, but smaller grape-like structures were apparent. Higher magnifications (See Figure 7) suggest that the larger clusters are more loosely associated than those in the KD background (compare e.g. with Fig. 6).

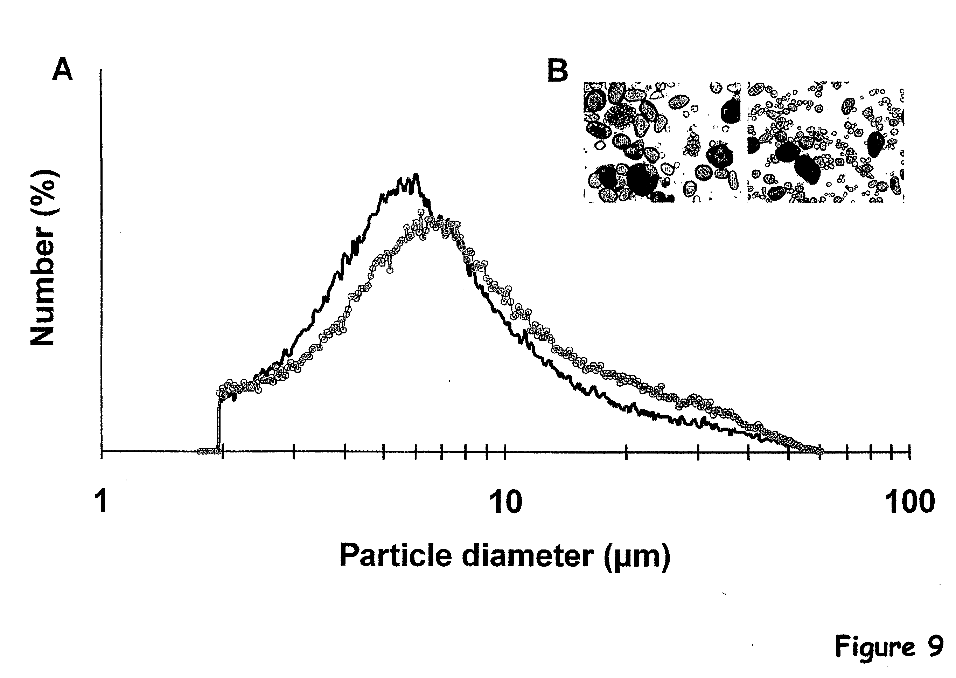

In order to investigate the cross-linking of grape-like starch granules, transgenic starches from *amf*SS3 were first treated with trypsin. The microscopic results suggest that trypsin could not separate the small units from grape-like structures (data not shown), suggesting that the clusters are not held together by protein. Subsequently, *amf*SS3 starches were treated with α-amylase. Samples without an α-amylase treatment served as a control. The forthcoming digest and control were dried. The morphology and granule size distribution of samples were measured. The results are shown in Figure 9. The grape-like structures were not observed in the α-amylase treated starch sample (Fig. 9B), indicating that small units in grape-like starch granules were separated. The granule size distribution of the sample with an α-amylase treatment showed a shift to smaller granule sizes in comparison with the control (Fig. 9A).

Also the surface of the granules appeared rough and contained deep grooves, particularly in the 6+ class. From these observations it was concluded that the grape-like structures could also be observed in an *amf* background, although the subunit particles seem to be more loosely associated. Further, a high level SBD2 accumulation was found to be associated with a rough and eroded granule surface. The experiments did not show an unambiguous relationship between the altered morphology and the level of SBD2 accumulation, although there seemed to be a tendency that the more severe alterations are associated with high levels of SBD2 accumulation.

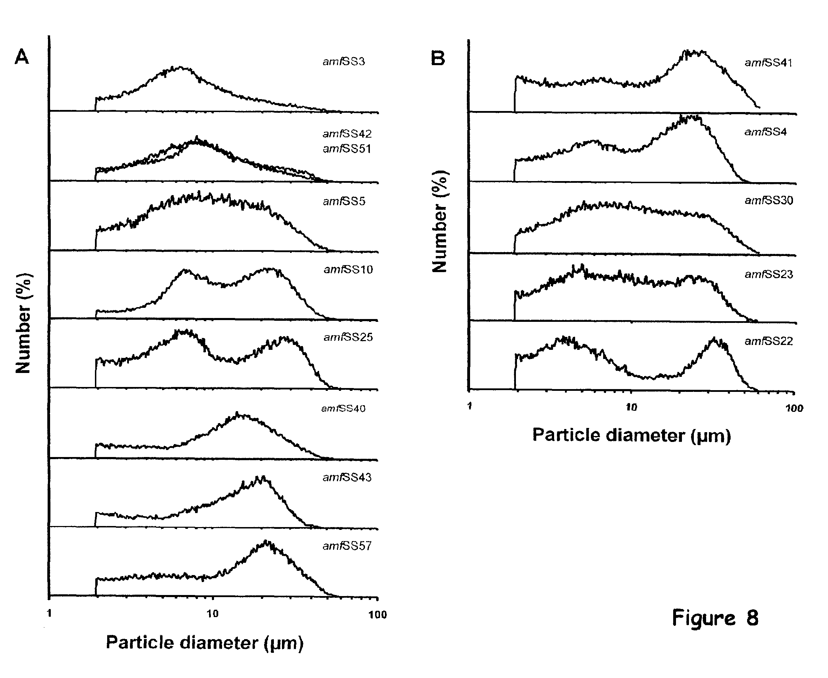

Following microscopic observations, the starches produced were subjected to size measurements. To this end, approximately 10 mg of starch was dispersed in 160 ml of Isoton II. The granule size distribution was recorded by counting 50,500 (± 500) particles in a Coulter Counter. Results of the various measurements are incorporated as Figure 8. The Figures 8 A and B show examples (3+ and 6+ class) of the differences in granule size distributions that could be encountered in SBD2 transgenic starches. Rheological studies on the starches were performed. These studies included measurement of the starch gelatinisation temperature, and the enthalpy released. The results are summarized in Table 1.

**Table 1.**

| Amount of SBD2 protein accumulated in starch granule and gelatinization characteristics (*T*₀, *ΔH*) measurements of starches from transgenic lines and controls | | | |
|---|---|---|---|
| Clone | Amount of SBD2 | *T*₀ (°C)^{a} | *ΔH* (kJ/g)^{b} |
| *amf-*UT | | 68.78 (± 0.2) | 8.72 (± 0.7) |
| *amf*SS1 | 1+ | 69.22 (± 0.1) | 11.60 (± 0.5) |
| *amf*SS46 | 2+ | 67.67 (± 0.2) | 9.68 (± 0.4) |
| *amf*SS3 | 3+ | 68.22 (± 0.1) | 9.45 (± 1.0) |
| *amf*SS55 | 4+ | 68.82 (± 0.2) | 16.53 (± 1.1) |
| *amf*SS34 | 5+ | 67.36 (± 0.1) | 12.21 (± 1.8) |
| *amf*SS23 | 6+ | 69.26 (± 0.2) | 11.56 (± 2.0) |

| | | | |
|---|---|---|---|
| ^{a} Temperature of onset of starch gelatinization. | | | |
| ^{b} Enthalpy released. | | | |

### Example 2.

### Preparation of constructs

Two constructs were made (see Fig. 1), one for SBD2 expression in *E. coli* [pTrcHisB/(SBD)₂], and one for SBD2 expression in potato plants [pBIN19/(SBD)₂]. The pTrcHisB/(SBD)₂ construct was assembled from the pTrcHisB/SBD plasmid (Ji *et al.,* 2003, Plant Mol. Biol. 51, 789-801). A sequence similar to SBD in pTrcHisB/SBD and an artificial PT-linker were inserted into pTrcHisB/SBD. The second SBD-encoding sequence was obtained by PCR amplification with the primers 5'-CAACTTCGAG**GAATTC**TACG-3' and 5'-**AAGCTT**ATGGCTGCCAATTCAC-3', which contain EcoRI and HindIII sites at their 5'-ends, respectively. The CGTase gene from *Bacillus circulans* strain 251 was used as a template (Lawson *et al.,* 1994). The amplified fragment was subcloned into a pGEMTeasy vector (Promega, USA). Plasmid DNA was propagated in *E. coli* DH5α and purified from the cells using the Wizard *Plus* Midipreps DNA purification system (Promega, USA). After digestion of this plasmid, the EcoRI-HindIII SBD fragment was inserted into the corresponding sites of the pTrcHisB/SBD expression vector to give the pTrcHisB/SBDI-SBDII vector. The PT-linker (Ji *et al.,* 2003) used to connect the two SBDs contained a BgIII site at 5'-end and an EcoRI site at 3'-end. The linker was inserted into the corresponding sites of the pTrcHisB/SBDI-SBDII vector to give the pTrcHisB/(SBD)₂ expression vector (see Fig. 1).

Using standard cloning procedures, the pBIN19/(SBD)₂ vector was assembled from four DNA fragments: (i) the potato tuber-specific GBSSI promoter (HindIII-NcoI) (van der Leij *et al.,* 1991), (ii) a sequence encoding the potato GBSSI transit peptide for amyloplast-entry (NcoI-NcoI) (van der Leij *et al.,* 1991), (iii) a SBD2 fragment (NcoI-BamHI), and (iv) the NOS terminator sequence (SacI-EcoRI) (Bevan, 1984). The first two fragments were subcloned in pMTL23 (ji *et al.,* 2003). The NcoI-BamHI SBD2 fragment was amplified by PCR with the primers 5'-**CCATGG**CCGGAGATCAG-3' and 5'-CTTCTC**GGATCC**GCCAAAAC-3' using pTrcHisB/(SBD)₂ as a template. The combined fragments (i)-(ii) and fragment (iii) were cloned in a pBIN19 vector, which already contained the NOS terminator sequence. The plasmid is referred to as pBIN19/(SBD)₂ (see Fig. 1). The splice site for cleavage of the transit peptide is the same as used previously for SBD targeting (Ji *et al.,* 2003). The SBD2 protein, which will be accumulated in potato tubers, has a predicted molecular weight of 25,371 Da, excluding transit peptide. Both constructs were sequenced to ensure no mutations had occurred during the cloning procedure.

### Plant transformation and regeneration

The pBIN19/(SBD)₂ plasmid was transformed into *Agrobacterium tumefaciens* according to the three-way mating protocol described by Visser (1991). Internodal stem segments from the amylose-free (*amf*) potato mutant (often referred to as 1029,31; Jacobsen *et al.,* 1989) were used for *Agrobacterium*-mediated transfomation containing the plasmid (Visser, 1991). More than 50 independent shoots were harvested. Shoots were tested for root growth on a kanamycin-containing (100mg/L) MS30 medium (Murashige and Skoog, 1962). Fifty transgenic, root-forming, shoots were multiplied and transferred to the greenhouse for tuber development. In addition, 10 untransformed controls were grown in the greenhouse.

### Isolation, and analysis of potato tuber starch

Potato tuber starch was isolated according to the method described by Ji et al. (2003). The amount of SBD2 protein accumulated in transgenic starches was estimated with a Western dot blot procedure as described by Ji et al. (2003). AntiSBD was used as a primary antibody. Starch granule morphology was investigated by light microscopy (LM, Axiophot, Germany). Starch granules were stained with a 20× diluted Lugol's solution (1% I₂/KI).

Average granule size and granule size distribution of the starches were determined in triplicate with a Coulter Multisizer II, equipped with an orifice tube of 100 µm (Beckman-Coulter, UK). Approximately 10 mg of starch was dispersed in 160 mL of Isoton II. The number percentages of the differently-sized granules in the sample were recorded by counting approximately 50,500 (±500) particles. The coincidence (the frequency of two granules entering the tube at the same time, and consequently being counted as one) was set at 10%.

The apparent amylose content of starches was determined according to the method described by Hovenkamp-Hermelink *et al.* (1989).

The temperature at which starch granules start to gelatinize was determined by differential scanning calorimetry (DSC) using a Perkin-Elmer Pyris 1 (Perkin-Elmer, The Netherlands), equipped with a Neslab RTE-140 glyco-cooler (Ji *et al.,* 2003). Dynamic rheological properties of 5% (w/v) starch suspensions at small deformations were determined by applying a small oscillating shear deformation (5 s⁻¹) using a Bohlin CVO rheometer (Mettler Toledo, The Netherlands). The suspensions were preheated to ∼40 °C under gentle stirring and loaded in the sample cell (preheated at 40 °C). After this, the cell was subjected to the following temperature program: heating to 90 °C, 15 min at 90 °C, cooling to 20 °C, and 15 min at 20 °C. Heating and cooling were performed at a rate of 2 °C/min. Date were collected automatically every 10s.

### Characterization of SBD2 transformants

The *amf* potato mutant was transformed with the pBIN19/(SBD)₂ construct. The forth-coming transgenic potato plants are referred to as *amf*SSxx (where SS represents the SBD2 gene and xx refers to the clone in the series of transformants). Untransformed control plants are referred to as *amf*-UT. Fifty kanamycin-resistant, transformed lines were grown in the greenhouse to generate tubers. During growth, the transgenic lines appeared phenotypically normal (results not shown).

The levels of SBD2 protein accumulation in transgenic granules were investigated by Western dot blot analysis. The SBD2 accumulating lines were grouped in 7 classes (ranging from 0+ to 6+), based on the amount of SBD2 protein that was associated with the starch granules (see Fig. 3B). The 6+ class (not shown in Fig. 3B) represents the transgenic granules, giving a similar signal in the Western dot blot analysis as the 5+ class, with half the amount of starch. SBD2 accumulation levels in the transgenic granules are summarized in figure 3A. For comparison, single SBD accumulation in the *amf* background *(amf*S series) is also indicated in this figure (Ji *et al.,* 2003). It is apparent that in the *amf*SS series many more transformants are found in the classes (4+, 5+, and 6+) with high SBD2 accumulation, whereas for the *amf*S series these classes are not observed at all. These results demonstrate that SBD2 is a higher-affinity binding domain than SBD.

In order to estimate the amount of SBD2 protein accumulated in transgenic granules, *amf*S48, the highest SBD accumulator (3+ class) from the *amf*S series (Ji *et al.,* 2003) was used as a control for comparison with the density of dots from the *amf*SS series in Western dot blot analysis. The amount of SBD2 in the highest accumulator (6+ class) was estimated to be approximately 5-fold higher than that of amfS48 (data not shown).

### Granule size is altered by SBD2 expression

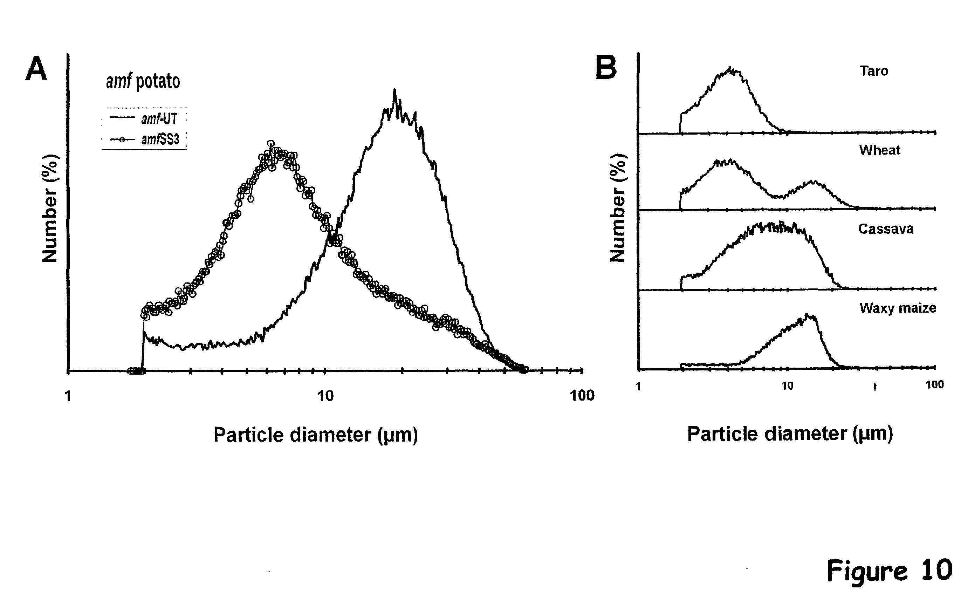

Microscopic examination of starch granule morphology from 50 transformants revealed that the size of the starch granules of some transgenic lines was substantially smaller than that of the control, particularly in the *amf*SS3 transformant. This line appeared to have the most dramatic reduction in granule size. This observation was further substantiated by measuring the granule size distribution of *amf*SS3 starch (Fig. 10A). Starch from *amf*-UT served as a control (Fig. 10A). The median (d₅₀) granule size by number of *amf*-UT starch was 17.1 µm, whereas the d₅₀ of *amf*SS3 starch was 7.3 µm, which is approximately 2.5 times smaller than the control.

For comparison, the granule size distributions of a number of important crop plants are shown in figure 10B. Taro starch has a d₅₀ of 4.1 µm, which is to our knowledge the smallest starch granule known in a crop plant. Wheat starch has a bimodal granule size distribution with large granules of the A-type crystallite and smaller ones with a B-type crystallite (French, 1984). The d₅₀ of wheat starch is 4.1 µm for the smaller granules, and 15.0 µm for the larger ones (Fig. 10B). Maize and cassava are major starch sources for the industry. The d₅₀ of maize starch is 11.2 µm, whereas that of cassava is 7.5 µm. Our results show that the granule size of amylose-free potato starch can be decreased to that of (wild-type) cassava by SBD2 expression.

### Starch gelatinization behaviour, pasting properties, and amylose content

The impact of SBD2 accumulation in granules on the physico-chemical properties of the transgenic starch was also investigated. Granule-melting behaviour (*T*₀ and *ΔH)* of the *amf*-UT and *amf*SS3 starch were studied by DSC. The pasting properties of the two starches were determined by Bohlin rheometry, which involves measuring viscosity changes while the starch suspension is heated and then cooled with constant stirring. The highest viscosity of a starch paste is referred to as the peak viscosity, whereas the viscosity after cooling of the starch paste is called the end viscosity. Also, the amylose content of both samples was analyzed. There were no consistent differences in the various parameters for the two starches. The same is true for the chain-length distribution of the two starches.

### Abbreviations:

- SBD: starch-binding domain
- GBSS I: granule-bound starch synthase
- KD: Kardal (potato cultivar)
- KD-UT: Kardal untransformed
- KDS: Kardal transformant containing SBD gene
- KDSS: Kardal transformant containing double SBD gene
- *amf*: amylose-free potato mutant
- *amf*-UT: amylose-free mutant untransformed
- *amf*S: *amf* mutant transfomant containing SBD gene
- *amf*SS: *amf* mutant transfomant containing double SBD gene

## Claims

1. Method for modifying the size and/or morphology of starch granules, which method comprises the production of a transgenic starch-producing plant, said plant being capable of producing a polypeptide comprising at least two fused starch-binding domains.

2. Method according to claim 1, wherein said transgenic plant is produced by a process comprising the steps of:
a) producing a polynucleotide construct comprising:
i) at least one promoter sequence which is active in said plant;
ii) at least two nucleotide sequences encoding a starch-binding domain;
b) transferring and incorporating the said polynucleotide construct into the genome of a cell of the said plant; and
c) regeneration of an intact, transgenic plant from the transformed plant cell.

3. Method according to claim 1 or 2, wherein the said starch-binding domains are heterologous to said plant

4. Method according to any one of the previous claims, wherein the said starch-binding domains are derived from family I SBDs of amylolytic enzymes.

5. Method according to any one of the previous claims, wherein the said starch-binding domains are fused via a linker.

6. Method according to claim 5, wherein said linker is selected from the 40 AA linker sequence used in *Trichoderma,* naturally occurring or synthetic sequences of alternating Pro-Thr and the linker sequences containing major amounts of glycine residues or of (optionally O-glycosylated) serine or threonine residues, preferably the PT-rich linker peptide derivable from exoglucanase (Cex) from *Cellulomomas fimi.*

7. Method according to any one of claims 2-6, wherein the said promoter is selected from the group consisting of CaMV promoter, GBSS promoter, patatin promoter, Ubiquitin promoter, ST1 promoter, TR1 promoter and napin promoter.

8. Method according to any one of claims 2-7, wherein said polynucleotide construct further comprises a signal sequence for plastid targeting.

9. Method according to any one of the previous claims, wherein said plant is potato (*Solanum tuberosum L.*).

10. Method according to any one of the previous claims, wherein said modifying the size of starch granules comprises the reduction of the mean starch granule size.

11. Method according to any one of the previous claims, wherein said modifying the size of starch granules comprises modifying the size distribution of the starch granules.

12. Polynucleotide construct comprising coding regions for at least two starch-binding domains.

13. Polynucleotide construct of claim 12, wherein said regions are connected by a linker sequence.

14. Vector comprising a polynucleotide construct according to any one of the claims 11 or 12.

15. An *Agrobacterium* strain or any other microbial strain comprising a vector according to claim 14.

16. Polypeptide construct comprising at least two fused starch-binding domains and which domains are optionally connected via a linker.

17. Method for producing a transgenic plant comprising introducing into the genome of said plant a vector according to claim 18.

18. Method according to claim 17, wherein said introducing comprises introducing said vector into an ancestor plant, and then producing said transgenic plant from said ancestor plant.

19. Method according to claim 18, wherein said introducing comprises introducing said vector into a plant part to produce a transformed plant part, and then regenerating said transgenic plant from said transformed plant part.

20. Transgenic plant or plant part produced by a method according to any of the claims 17-19.

21. Transgenic starch-producing plant or plant part, wherein the size and/or morphology of starch granules is modified and which plant or plant part is capable of producing a polypeptide comprising at least two fused starch-binding domains.

22. Use of a plant according to claims 20 or 21 for the production of size-and/or morphology-modified starch.

23. Size- and/or morphology-modified starch obtainable by a method according to any one of the claims 1-11.

24. Starch according to claim 23, wherein said starch is additionally cross-linked, carboxymethylated or hydroxypropylated.

25. Potato starch where the weight average particle size of the starch granules is lower than 25 µm, preferably less than 20 µm.

26. Use of a starch according to any one of the claims 23 to 25, to improve viscosity, texture, mouthfeel, taste, processability and/or stability of a food product.

27. Use of a starch according to any one of the claims 23 to 25, in soups, sauces, gravies, ready-to-serve desserts, low-fat yoghurts, bakery fillings, jams, marmalades, canned fish, canned meat, snacks, noodles and/or baked goods.

28. Use of a starch according to any one of the claims 23 to 25, in printing ink formulations, in adhesives, in drilling fluids, as a filler and/or as a slow-release carrier.
